Europäisches Patentamt

European Patent Office

Office européen des brevets

(19)

(11) Publication number: **0 117 117**
**A1**

(12) # EUROPEAN PATENT APPLICATION

(21) Application number: **84300937.4**

(22) Date of filing: **14.02.84**

(51) Int. Cl.³: **A 61 K 31/565**

(30) Priority: **22.02.83 US 468519**

(43) Date of publication of application:
**29.08.84 Bulletin 84/35**

(84) Designated Contracting States:
**AT BE CH DE FR GB IT LI LU NL SE**

(71) Applicant: **Liehr, Joachim Georg**
**115 Beverly Lane**
**Bellaire Texas(US)**

(72) Inventor: **Liehr, Joachim Georg**
**115 Beverly Lane**
**Bellaire Texas(US)**

(74) Representative: **Tapping, Kenneth George et al,**
**Lilly Industries Limited Patent Department Erl Wood**
**Manor**
**Windlesham Surrey, GU20 6PH(GB)**

(54) Improvements in or relating to the treatment of estrogen deficiency conditions.

(57) 2-Fluoroestradiol may be used to treat estrogen-deficiency states in human females who have a predisposition to cancer of estrogen sensitive tissue.

EP 0 117 117 A1

X-6094                          -1-

## IMPROVEMENTS IN OR RELATING TO THE TREATMENT OF ESTROGEN DEFICIENCY CONDITIONS

This invention relates to 2-fluoroestradiol and its use in the treatment of estrogen deficiency conditions.

Prolonged treatment of experimental animals with exogenous estrogens including steroid hormones such as estrone, estradiol and estriol, has been reported to induce tumors in the testes, endometrium, ovary, cervix and lymphoid tissues, mammary tissue, adrenal cortex, kidney and anterior pituitary gland. Synthetic estrogenic compounds such as DES also have been found to increase incidence of tumors in experimental animals.

The exact mechanism of tumor induction by these hormones is unknown. Some researchers have stated that the role of estrogens on the etiology of breast cancer and other gynecologic cancers may be permissive rather than causative. Various epidemiological studies indicate that use of estrogen by post-menopausal women is associated with an increased risk of endometrial cancer [Goodman and Gilman - The Pharmacological Basis of Therapeutics, 6th Ed. - pp. 1426, 1429-30 (McMillan, New York 1980)].

In this regard, researchers have proposed that the ring A polyhydroxylated estrogens, the primary products of estradiol or estrone metabolism which are almost devoid of estrogenic activity, could be responsible for the carcinogenic or carcinopromoting action of estradiol. These polyhydroxy metabolites, 2- and 4-hydroxyestradiol or 2- and 4-hydroxyestrone, (catechols or ortho-dihydroxybenzenes) are the major biotransformation products of estradiol in most species and are known to bind covalently in vitro to protein or peptides and DNA. One should note that such binding to protein can be suppressed by prior methylation of the catechol.

If oxidation of the phenolic ring of an estrogen is indeed a necessary step leading to increased numbers of tumors in experimental animals, then interference with metabolic oxidation should inhibit cancer induction by estrogens. Following this hypothesis, Li and Li, Proc. 72nd Annual Meeting Am. Assoc. Can. Res., 22, 11 (1981), were able to inhibit, by chronic administration of inhibitors of microsomal cytochrome P-450 enzymes (oxidation enzymes), diethylstilbestrol-induced renal carcinoma in Syrian hamsters. Another means to prevent carcinogenesis by estrogens would be to modify the estrogen structure to prevent oxidation to a catechol.

In this regard, research has shown that the tumorigenicity of dibenzo[a,i]pyrene has been reduced by fluorination at positions 2,3 and/or 10. Oxidation of the hydrocarbon ring to a catechol has been implicated in tumor production by this compound.

A genetic predisposition to cancer of estrogen dependent tissues in human females apparently exists because a history of breast cancer in first degree relatives increases the risk of such cancer 2-3 times [CMA Journal, 121, 505-8 (1979)]. In such circumstances, the increased risk of induced cancer by administration of estrogen for treatment of estrogen-deficiency states weighs heavily against such therapy. Stated simply, a woman, fearful of incurring cancer because of a history of greater than normal cancers, in her relatives, of the breast, cervix, uterus or ovaries will have to endure, in many instances, menopause rather than being able to alleviate by taking an estrogen, hot flashes and other menopausal symptoms. This invention is directed to this group of woman, i.e., those with a genetic predisposition to cancer of estrogen sensitive tissues.

Thus, this invention provides an estrogen of decreased cancer induction potential, which is useful in treating menopause or other estrogen deficiency states in human females.

In particular, there is provided a method for alleviating the symptoms of estrogen-deficiency conditions in human females, particularly those having a genetic predisposition to cancer of estrogen sensitive tissues which comprises administering to the female, 2-fluoroestradiol. Human females within this category may be those in whose first degree relatives one or more cancers of estrogen sensitive tissues exist. First degree relatives include parents, siblings and children.

The most prevalent estrogen-deficiency condition is menopause which can occur naturally or can be induced by destruction of ovarian function, either surgically or by irradiation. Because menopausal symptoms are not only unpleasant but may include osteoporosis, menopause commonly is treated by making up the estrogen deficiency through administration of estrogen. Commonly prescribed estrogens for alleviation of menopausal symptoms include DES (diethylstilbestrol), conjugated forms of estrogens such as equilin, estrone etc. ethinyl estradiol, hexestrol and dienestrol. Obviously, an estrogen with lowered potential for inducing cancer of estrogen sensitive tissues would be invaluable, particularly for those human females with a genetic predisposition to such neoplasms.

The following experiments were carried out to demonstrate the decreased incidence of estrogen-induced tumors with 2-fluoroestradiol as compared to estradiol. First, the estrogenicity of these compounds, in addition to that of isomeric 4-fluoroestradiol, were measured by two different methods. In one method, the increase in uterine weight of ovariectomized, immature rats, after administration of estrogen, was determined. The results of this test show that the fluorinated compounds are approximately as estrogenic as estradiol. The following protocol was used:

Female Sprague/Dawley rats, 3-4 weeks of age, were ovariectomized. Four days after ovariectomy, animals were separated into four groups of equal group weight (17 animals/group, average weight: 90 $\pm$ 1 g/animal) and estrogen treatment was begun. The animals received s.c. injections of 3.5 µg of estrogen, in 0.7 ml of saline/animal, once daily for three days. Saline solutions were prepared by dissolving the estrogen in ethanol and diluting this solution with saline until a 5% ethanol/95% saline mixture was obtained. The control group received only injections of saline. On the fourth day, the animals were killed, the uteri were excised, freed of luminal fluid, and weighed. Data presented in Table 1 are averages of uterine weights (in mg) and the corresponding calculated standard deviations.

In a second method, the weight of both testes of the male Syrian hamsters also used in the in vivo carcinogenicity assay described later, was measured on the 224th day after s.c. implantation of 25 mg pellets of estrogen. The weights were measured in grams and are expressed as percent of total body weight. Treatment of animals with modified estrogens or with estradiol resulted in the shrinking of the testes to approximately 10% of the weights of testes in untreated control group of hamsters.

The following protocol was employed:

Male Syrian hamsters, 3-4 weeks of age (Harlan/Sprague Dawley, Madison, Wisconsin), were given a 25 mg s.c. implant consisting of 10% cholesterol and 90% estrogen. A second 25 mg pellet of the same composition was implanted 106 days after the initial estrogen treatment. Estrogen treatment was carried out according to the procedure of Kirkman, National Cancer Institute Monograph, 2, 1-57 (1959). Control animals were not treated. Two hundred twenty-four days after treatment began, the hamsters were weighed, killed, both testes were excised and their weights measured in grams. The data presented in Table 1 are the ranges of testes weights, expressed as percentages of total body weights, from four animals in each group.

In Table 1, column 1 gives the name of the compound tested, column 2 the weights of testes of the male hansters (as percent of total body weight), and column 3 the uterine weights found.

### Table 1
### Estrogenic Activity of Estradiols

| Compound | Range of testes weight of male hamsters [% of total body weight] | Female Rat Uterine weights [mg] ± s.d. |
|---|---|---|
| 2-fluoroestradiol | 0.18 - 0.25 | 92 - 20 |
| 4-fluoroestradiol | 0.18 - 0.28 | 118 - 19 |
| estradiol | 0.12 - 0.26 | 114 - 20 |
| control | 1.85 - 2.06 | 33 - 6 |

These estrogenicity results compare well with experiments reported by Heiman et al., J. Med. Chem., 23, 994 (1980), who reported the estrogen receptor binding affinities of the fluorinated estrogens. When expressed as ratios of association constants ($K_a^{compound}$/$K_a^{estradiol}$ x 100), the binding affinity of 2-fluoroestradiol was found to be 86 and that of 4-fluoroestradiol was 128.

Further, Pfeiffer et al., Proc. Soc. Neuroscience, 8, 52 (1982), measured the estrogen receptor affinity of 2- and 4-fluoroestradiol in cytosol of the hypothalamus-preoptic area, the pituitary, and the uterus. The fluorinated estradiols were found to have high receptor affinity. Furthermore, biological responses of the fluorinated estrogens, such as elicitation of luteinizing hormone surges, of proceptivity (i.e., expression of female's sexual interest in response to stimuli from males; see, F.A. Beach, Hormones and Behavior, 7, 105 (1976)), and of lordosis behavior in rats were, according to MacLusky et al, ibid, and Krey et al., Fogarty International Conference (in press), comparable with those elicited by estradiol.

The carcinogenicity of the same estrogens was measured in male Syrian hamsters in vivo. Estrogen alone causes renal clear cell carcinoma in 100% of the male animal population when exposed to s.c. estrogen implants. This kidney tumor is estrogen-induced and estrogen-dependent for development and growth. Failure to resupply estrogen every three months or surgical removal of the implants results in tumor regression within a few weeks. Similarly, a tumor cell line derived from the Syrian hamster renal clear cell carcinoma was found to be strongly estrogen dependent in vivo, showing a 90% tumor regression 10 days after removal of the source of estrogen. The spontaneous kidney tumor incidence in Syrian hamsters without estrogen treatment is very low. The above facts are discussed in Kirkman, Nat'l. Can. Inst. Monograph, 1, 1 (1959); McGregor et al., J. Nat'l. Can. Inst., 24, 1057 (1960) and Sirbasku et al., Endocrin., 98, 1260 (1976).

X-6094                                   -8-

The protocol used to determine carcinogenicity was that set out for determining estrogen activity in male hamsters according to Table 1 (<u>i.e.</u>, the animals examined for tumors at 224 days were the same as the source of the testes). In the carcinogenicity experiment, further groups of animals also were examined at 279 and 345 days after treatment began. The animals were decapitated, the kidneys were excised and inspected visually for the occurrence of renal clear-cell carcinoma. Sections were prepared of all kidneys and studied histologically. The results of the histologic examinations are shown in Table 2. In the table, column 1 gives the name of the test compound, column 2 gives the total number of animals screened, column 3, the number of dead animals and columns 4, 5 and 6 give the results of histologic examination for renal clear-cell carcinoma at the specified time interval.

X-6094 -9-

## Table 2

In vivo Carcinogenicity of Modified Estrogens

| Compound | No. of Animals | Number of dead animals* | No. of animals with tumors/ No. of animals examined on various days after s.c. implantation of estrogen | | |
|---|---|---|---|---|---|
| | | | 224 Days | 279 Days | 345 Days |
| Estradiol | 18 | 5 | 4/4 | 5/5 | 0/4 |
| 2-fluoro-estradiol | 15 | 3 | 0/4 | 0/4 | 0/3 |
| 4-fluoro-estradiol | 15 | 2 | 1/4** | 3/6 | 0/3 |
| control | 10 | 0 | 0/3 | 0/3 | 0/4 |

*Number of dead animals included those which died from various causes during the course of the experiment. Tumors appear approximately six months after pelleting with estrogen. This group includes all hamsters found dead during the first six months of the experiment, since tumors were not expected and not evident in the kidneys of these animals. Hamsters found dead after six months were also included in this group if their kidneys could not be recovered due to tissue degeneration. One dead hamster with recoverable kidneys was included in the nearest examination group at 279 days (animal treated with 4-fluoroestradiol without renal carcinoma).

**2 of 4 judged histologically to be preneoplastic.

Table 2 clearly shows that 2-fluoroestradiol has a greatly decreased potential for induced renal clear cell carcinomas compared with estradiol or 4-fluoroestradiol at equal estrogen dosages. Mere blocking of the ortho oxidation of estradiol to a catechol, as in 4-fluoroestradiol, is insufficient by itself to eliminate estrogen-induced renal clear cell carcinomas in hamsters. This shows that 2-fluoroestradiol is highly estrogenic yet non-carcinogenic in the particular animal model. These findings are unique in that this represents the first time that separation of estrogenicity and carcinogenicity of estrogenic hormones has been shown.

The data observed at 345 days indicates that circulating estrogen had decreased, and perhaps returned to its normal untreated baseline concentration, and that regression of the previous tumors occurred as described earlier. Such results are not surprising in view of the previously described research showing the high estrogen-dependency of these tumors.

The fluoroestradiols used in this study were synthesized according to the procedure of Utne et al., J. Org. Chem., 33, 2469 (1968).

Estrogens, such as 2-fluoroestradiol of the present application, commonly are administered not only in the free form, but also in the form of pharmaceutically-acceptable esters or ethers. "Pharmaceutically-acceptable esters and ethers" are those useful in hormone replacement therapy and may include, without limitation, the acetate, benzoate, propionate, hemisuccinate and sodium hemisuccinate, heptanoate, undecanoate, caproate, valerate, enanthoate, sulfate and salts of sulfates, phosphate and salts of phosphates, cyclopentanepropionates ("cypionate"), methyl and ethyl ethers, as well as glycosidic ethers such as the glucoside, glucoside acetates, maltoside, and maltoside acetates. In addition, the 2-fluoroestradiol molecule possesses two free hydroxy groups, either or both of which may be etherified or esterified. Consequently, pharmaceutically-acceptable esters or ethers includes the di-esters and di-ethers. Examples of di-substituted derivatives may include, for example, the diacetate, dipropionate, and bis-(cyclopentanepropionate).

In the treatment of estrogen deficiency states such as menopause, 2-fluoroestradiol can be formulated for oral administration usually in the form of tablets or capsules containing from .10 to 25 mg, and preferably from 1-2 mg, of drug per tablet. A typical dosage for the treatment of menopausal symptoms may be from about 1-2 mg per day, although the dose can be increased or decreased as necessary for the individual.

Further, these estrogens may be administered parenterally. In such a case, 2-fluoroestradiol, or a pharmaceutically-acceptable ester or ether thereof, may be dissolved in a physiologically-acceptable fluid. Examples of such physiologically-acceptable fluids may include water for injection, isotonic normal saline or other suitable diluents recognized by those skilled in the art.

X-6094                                    -12-

   Other known means of administering estrogens
may be suitable for 2-fluoroestradiol as well.  Such
means may include slow release implants, slow release
intramuscular depot injections, vaginal inserts, pellets
or other means recognized by those skilled in the art.

## CLAIMS

1.   2-Fluoroestradiol, or a pharmaceutically-acceptable ester or ether thereof, for use in alleviating symptoms of estrogen deficiency conditions of human females.

2.   A compound as claimed in claim 1 in which the estrogen-deficiency condition is menopause.

3.   A compound as claimed in claim 1 or 2 in which the human female has an inherited predisposition to cancer of estrogen receptive tissues.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | JOURNAL OF ORGANIC CHEMISTRY, vol. 33, no. 6, June 1968, pages 2469-2473, Washington, D.C., US T. UTNE et al.: "The synthesis of 2- and 4-fluoroestradiol" * Page 2469, footnote 2; page 2470, compound VIIa; page 2472 * | 1-3 | A 61 K 31/565 |
| X | FR-A-1 519 508 (ROUSSEL-UCLAF) * Page 1 * | 1-3 | |
| P,X | CHEMCIAL ABSTRACTS, vol. 98, no. 19, 9th May 1983, page 57, no. 155364u, Columbus, Ohio, US J.G. LIEHR: "2-fluoroestradiol: separation of estrogenicity from carcinogenicity" & MOL. PHARMACOL. 1983, 23(2), 278-81 * Abstract * | 1-3 | |

TECHNICAL FIELDS SEARCHED (Int. Cl. ³)

A 61 K 31/00

The present search report has been drawn up for all claims

| Place of search | Date of completion of the search | Examiner |
|---|---|---|
| THE HAGUE | 12-05-1984 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82